# EUROPÄISCHE PATENTANMELDUNG

(11) **EP 1 769 822 A2**
(43) Veröffentlichungstag der Anmeldung: **04.04.2007**
(21) Anmeldenummer: 06025837.3
(22) Anmeldetag: 19.08.2002
(51) Int. Cl.: A61N 1/30

(54) **Iontophorese-Kaltkontaktelektrodensystem**

(30) Priorität: 23.08.2001 DE 10141254
(62) Teilanmeldung aus: 02755000.3
(71) Anmelder: Edel, Susann, 82031 Grünwald (DE)
(72) Erfinder: Edel, Susann, 82031 Grünwald (DE); Winkler, Rosemarie, 82377 Penzberg (DE)
(74) Vertreter: Banzer, Hans-Jörg

(57) **Zusammenfassung**

Ein lontophorese-Kaltkontaktelektrodensystem mit einem Elektrodenkörper (11) und einer Elektrodenoberfläche (13) zum Einbringen von iontophoretischen Wirkstoffen in die menschliche Haut und zum gleichzeitigen Kühlen der menschlichen Haut, um die elektrische Leitfähigkeit der Haut und damit das Einbringen der iontophoretischen Wirkstoffe in die Haut zu verbessern. Die Elektrodenoberfläche (13) kann wahlweise definiert gekühlt oder definiert erwärmt werden, wobei dies durch ein Peltier-Element (30) durch Umschalten der Polarität am Peltier-Element (30) erfolgen kann.

## Beschreibung

Die vorliegende Erfindung betrifft ein lontophorese-Kaltkontaktelektrodensystem mit einer definiert gekühlten Elektrodenoberfläche für den gleichzeitigen Vorgang der lontophorese-vorbehandlung durch das Abradieren der gekühlten, trockenen, verhornten schlecht stromleitenden Hautschichten und der optimierten lontophorese-Behandlung durch das verbesserte Eindringen des lontophorese-Stromes in die gekühlte und besser stromleitende Hautmembran zum iontophoretischen Einbringen von kosmetischen oder medizinischen Wirkstoffen über die menschliche Hautmembran in das darunterliegende Körpergewebe.

Die seit vielen Jahren angewendete lontophorese ist eine Methode, um elektrisch geladene, also ionisierte kosmetische oder medizinische Wirkstoffe über die Oberhaut in das darunterliegende Körpergewebe einzubringen. Dabei wird die elektrisch geladene Wirkstoffmischung entweder direkt oder mittelbar in einem Wirkstoffträger auf die Anwenderelektrode, genannt Kontaktelektrode, gebracht, welche dann an der gewünschten Stelle mit der Haut kontaktiert wird, wobei nur die Wirkstoffmischung oder der Wirkstoffträger nicht aber die Elektrode selbst mit der Haut in Berührung kommen darf. Die Gegenelektrode, genannt Groundelektrode, wird an einer anderen Hautpartie kontaktiert. Zwischen den beiden Elektroden wird nun eine elektrische Spannung angelegt, welche ein elektrisches Feld erzeugt, so dass ein sogenannter lontophoresestrom durch den Körper fließt, der die Wirkstoffmolekülionen durch die Haut transportiert. Die elektrische Ladung des Wirkstoffes bestimmt dabei die Polarität an den beiden Elektroden. Beispielsweise ist bei positiver Wirkstoff-Ladung die Kontaktelektrode positiv und die Groundelektrode negativ gepolt, so dass die positiv geladenen Wirkstoffmoleküle von der Kontaktelektrode weg in Richtung der Groundelektrode durch die Oberhaut in das darunterliegende Gewebe transportiert werden.

Wie bereits in der Patentschrift DE 694 25 728 T2 erwähnt, kann durch Auflegen von Eis- oder Wärmebeuteln die elektrische Leitfähigkeit der Haut beeinflußt werden. Das Auflegen der Kühl- oder Wärmebeutel wäre jedoch nur jeweils eine Vor- oder Nachkonditionierung mit kurzzeitiger Wirkung auf die Haut und dem zusätzlichen Nachteil, dass diese bisherige Technik ein schnelles Umschalten auf Heizen oder Kühlen sowie einen dauerhaften Einfluss auf die Hautoberflächentemperatur unmittelbar bei Ablauf der Behandlung nicht ermöglicht.

Seit vielen Jahren ist auch bekannt, dass eine Verbesserung der lontophorese bzw. des Eindringverhaltens bestimmter Wirkstoffe in die Haut durch starkes Vorkühlen der Haut erzielt wird. Zum einen dadurch, dass die trockenen, verhornten, elektrisch schlecht leitfähigen Hautschichten nach einer Kühlung besser abradiert werden können und zum anderen dadurch, dass der lontophoresestrom in die gekühlte und dadurch elektrisch leitfähigere Hautmembran besser eindringt. Erzielt wird die Vorkühlung der Haut bisher durch Eispackungen oder andere Kältepackungen, die vor der eigentlichen lontophoresebehandlung auf die jeweilige Hautpartie aufgelegt werden. Der Nachteil dieser Methoden ist, dass mehrere Vorgänge durchgeführt werden müssen und dass die Vorkühlung der Haut schnell nachlässt und somit der erzielbare Kühleffekt während der lontophorese kaum noch anhält und auch kein Abrasionseffekt erzielt werden kann.

Der vorliegenden Erfindung liegt die Aufgabe zugrunde, ein lontophorese-Elektrodensystem bereitzustellen, welches die zuvor beschriebenen Nachteile beseitigt und eine verbesserte lontophorese ermöglicht. Diese Aufgabe wird erfindungsgemäß durch ein lontophorese-Kaltkontaktelektrodensystem mit den Merkmalen des Anspruchs 1 gelöst. Die Unteransprüche definieren jeweils vorteilhafte und bevorzugte Ausführungsformen der Erfindung.

Bisher noch nicht bekannt war ein lontophorese-Elektrodensystem, das gleichzeitige lontophorese und Hautkühlung vereint. Die vorliegende Erfindung stellt eine bewegliche lontophorese-Kaltkontaktelektrode bereit, welche mit einfachen Mitteln die lontophorese ermöglicht und gleichzeitig die Haut kühlt. Wobei insbesondere der leichte Wechsel des mit einer abrasiven Oberfläche versehenen Wirkstoffträgers, genannt Wirkstoff-Pad, die feste Fixierung des Wirkstoff-Pad auf der Elektrodenfläche, die sehr gute Reinigungsmöglichkeit der Elektrode von Wirkstoffresten nach der Anwendung und insgesamt die unproblematische Handhabung in der Anwendung gewährleistet ist. Die vorliegende lontophorese-Kaltkontaktelektrode ist darüber hinaus insbesondere in der kosmetischen Anwendung durch die besondere Elektrodenform für die verschiedenen schwierigen Hautbereiche im Gesicht geeignet, und vor allem auch für den Anwender bei der Behandlung leicht anwendbar.

Das erfindungsgemäße lontophorese-Kaltkontaktelektrodensystem umfasst einen Elektrodenkörper mit einer innen gekühlten und derart nach außen erhabenen, gewölbten elektrisch leitenden Elektodenoberfläche, dass durch diese erhabene, gewölbte Form auch die engen Gesichtspartien um Nase, Mund, Augen und Ohren gut erreicht werden können. Bei Bedarf kann das Kühlelement im Inneren des Elektrodenkörpers durch Umschalten am Steuergerät auch als Heizelement zum Wiedererwärmen der Haut auf die normale Körpertemperatur verwendet werden. Dieser KaltANarm-Effekt dient zusätzlich der Hautkonditionierung.

Des weiteren umfasst das erfindungsgemäße lontophorese-Kaltkontaktelektrodensystem einen Elektrodendeckel mit einer derartigen mittigen großen Öffnung, dass der Elektrodendeckel mit seinem Deckelsteg den vorher auf die Elektrodenfläche aufgelegten, etwas überstehenden Wirkstoff-Pad beim Aufschrauben auf den Elektrodenkörper so auf der Elektodenfläche ganzflächig festklemmt, dass der Wirkstoff-Pad fest fixiert aus der Deckelöffnung herausragt.

Ebenso umfasst das erfindungsgemäße lontophorese-Kaltkontaktelektrodensystem einen Wirkstoff-Pad aus einem flexiblen, porösen, an der Oberfläche entsprechend stark aufgerauhten elektrisch nicht leitenden Material, der mit der elektrisch geladenen kosmetischen oder medizinischen Wirkstoffmischung, ähnlich einem vollgesogenen Schwamm, getränkt ist. Vorteilhafterweise wird der Wirkstoff-Pad als Wirkstoff-Einwegpad in luftdichter Verpackung gelagert und erst unmittelbar vor der Anwendung auf die Elektrodenfläche aufgelegt und wie oben beschrieben für die Behandlung fixiert. Die in einem Wirkstoff-Einwegpad enthaltene Wirkstoffmenge ist dabei gerade für eine Behandlung bemessen, so dass nach Ende der Behandlung der aufgebrauchte Pad entsorgt werden kann und die Elektrode für eine spätere neue Behandlung gereinigt werden kann.

Erläuterung der Figuren:

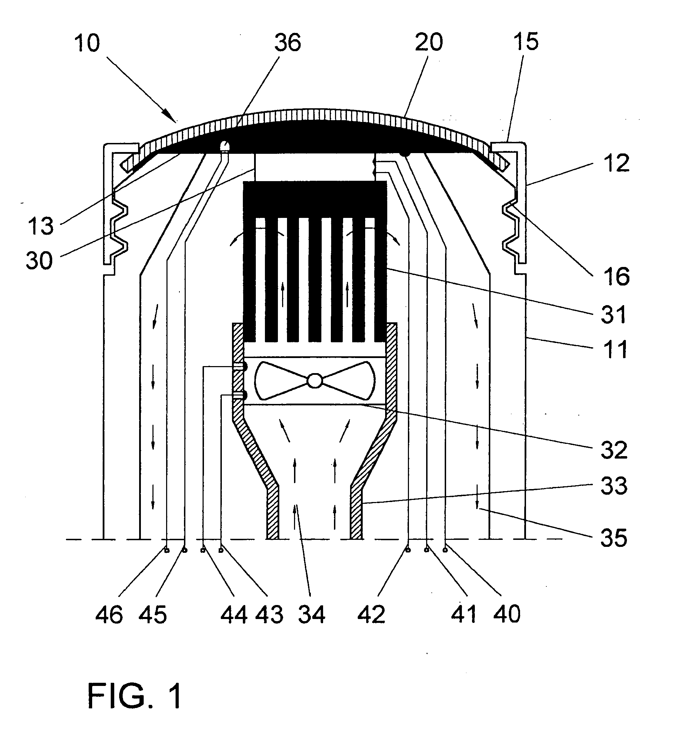

Figur 1 zeigt als Querschnittszeichnung beispielhaft ein erfindungsgemäßes lontophorese-Kaltkontaktelektrodensystem (10), bestehend aus dem Elektrodenkörper (11) mit der nach oben gewölbten elektrisch leitenden Elektrodenoberfläche (13) und dem aufgesetzten Wirkstoff-Pad (20), welches durch den Deckelsteg (15) des oben offenen Elektrodendeckels (12) auf die nach oben gewölbte Elektrodenoberfläche (13) flächig festgeklemmt ist und erhaben über den Elektrodendeckel (12) herausragt. Das Festklemmen des Wirkstoff-Pads (20) durch den Deckelsteg (15) wird durch das Festschrauben des Elektrodendeckels (12) mit seinem Innengewinde (16) auf das Außengewinde des Elektrodenkörpers (11) erreicht. Der rückseitige Abschluß des Elektrodenkörpers ist symbolisch als Strichlinie dargestellt.

Die Elektrodenoberfläche (13) wird mit einem, an ihrer Innenseite angebrachten elektronischen Kühl/Heizelement, vorzugsweise einem handelsüblichen Peltier-Element (30), definiert gekühlt bzw. geheizt, wenn dies zur besseren Konditionierung der Haut für das Einbringen der iontophoretischen Wirkstoffe vorteilhaft ist.

Bei Kühlfunktion erkaltet das Peltierelement (30) an der Elektrodenseite und nimmt so die Wärme von der Elektrodenoberfläche (13) auf und gibt sie auf seiner anderen Seite an einen dort angebrachten Kühlkörper (31) ab, dessen Kühlrippen durch ein Luftkühlsystem gekühlt werden. Bei diesem Luftkühlsystem saugt der vor dem Kühlkörper (31) angeordnete Ventilator (32) die kühle Außenluft (34) durch die Luftleithutze (33) an und bläst durch die Kühlrippen des Kühlkörpers (31) hindurch die erwärmte Abluft (35) durch den Innenraum des Elektrodenkörpers (11) nach Außen ab.
Bei Heizfunktion wird die Stromrichtung im Peltierelement (30) umgepolt, dabei erwärmt sich das Peltierelement an der Elektrodenseite und erwärmt so die Elektrodenoberfläche (13). Das Luftkühlsystem kann hierbei zur Temperaturregelung mit verwendet werden.

Die Temperatur der Elektrodenoberfläche (13) und damit auch die des aufgeklemmten Wirkstoffpads (20) wird durch den in die Elektrodenoberfläche (13) montierten Temperatursensor (36) ermittelt und als Temperatur-Istwert einem Temperaturregler in dem lontophorese-Steuergerät zugeführt, welcher die gewünschte Kühl- bzw. Heiztemperatur exakt regelt. Die Leitungen (40)- (46) stellen die Anschlußleitungen der elektrischen Komponenten des Kaltkontakt-Elektrodensystems mit dem lontophorese-Steuergerät dar, wobei Leitung (40) an der Elektrodenoberfläche (13) angeschlossen ist und den lontophoresestrom führt, die Leitungen (41-42) leiten den Strom zum Peltierelement (30), die Leitungen (43-44) versorgen den Ventilator (32) und die Leitungen (45-46) stellen die Verbindung zu dem Temperatursensor (36) her.

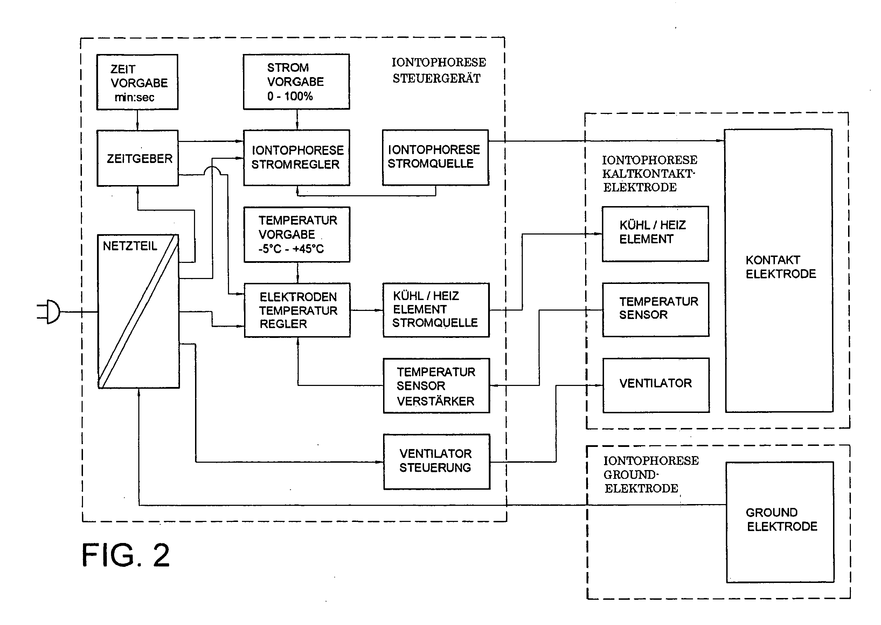

Figur 2 zeigt als Blockschaltplan beispielhaft einen Aufbau eines lontophoresegerätes mit einem erfindungsgemäßen lontophorese-Kaltkontaktelektrodensystem, der lontophorese-Groundelektrode und dem lontophorese-Steuergerät. Der lontophoresestrom wird im Steuergerät durch die Stromvorgabe in der Intensität und durch die Zeitvorgabe im zeitlichen Verlauf durch den lontophoresestromregler exakt geregelt und fließt, getrieben durch die lontophoresestromquelle, in die Oberfläche der lontophorese-Kaltkontaktelektrode, weiter durch den aufgeklemmten lontophorese-Wirkstoffpad in die Patientenhautpartie und weiter durch den Patientenkörper zu der an einer anderen Körperstelle kontaktierten Groundelektrode und dann zurück zum Netzteil im Steuergerät.

Die Kühlung bzw. Heizung der Oberfläche der lontophorese-Kaltkontaktelektrode wird durch ein Kühl-Heizelement ermöglicht, welches an einen Temperaturregelkreis, bestehend aus Temperaturvorgabe, Elektrodentemperaturregler, umpolbarer Stromquelle und einem an der Kontaktelektrode montierten Temperatursensor, angeschlossen ist. Der für die Luftkühlung des Kühlkörpers erforderliche Ventilator wird durch die Ventilatorsteuerung im Steuergerät angesteuert.

Zur Kühlung/Erwärmung der Elektrodenoberfläche 13 kann auch ein daran vorbei geleitetes und von einem entsprechenden Leitungssystem bereitgestelltes gekühltes/erwärmtes und vorzugsweise flüssiges Mittel, insbesondere ein Gas, verwendet werden.

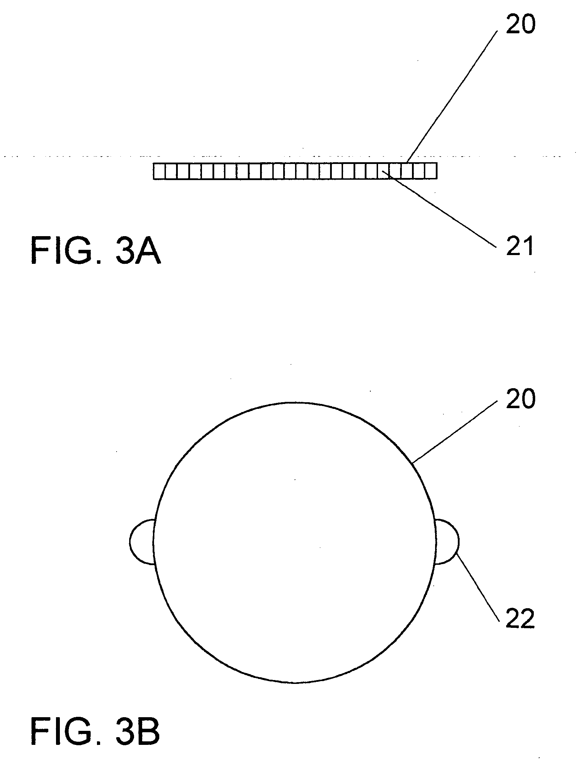

Figur 3A zeigt als Querschnittszeichnung beispielhaft einen mit dem iontophoretischen Wirkstoff (21) getränkten Wirkstoff-Einwegpad (20) aus einem saugfähigen porösen an der Oberfläche für die gewünschte Hautabrasion entsprechend stark aufgerauhten Material. Das Wirkstoff-Einwegpad (20) sollte wegen der besseren Haltbarkeit und der einfachen Anwendung in Einfach-Blister luftdicht verpackt sein, und als Einweg-Pad nach einer Behandlung entsorgt werden.

Figur 3B zeigt ein Wirkstoff-Einwegpad (20) beispielhaft mit zusätzlichen Ansätzen (22) zur leichteren Entnahme aus der Verpackung.

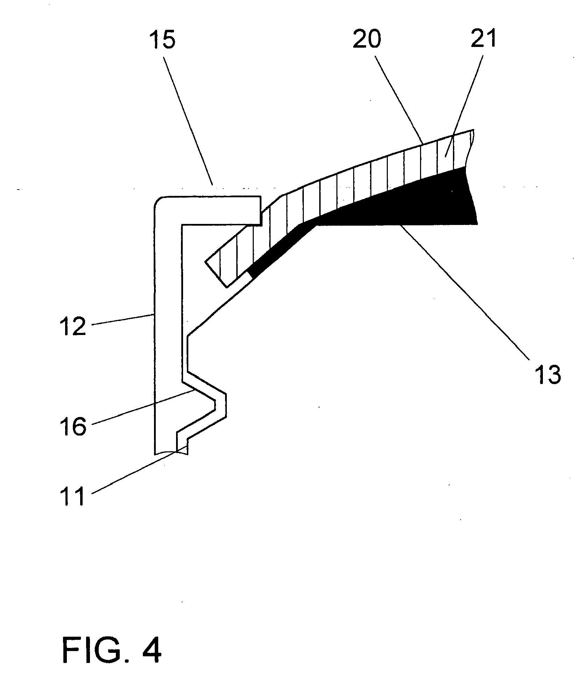

Figur 4 zeigt eine Querschnittszeichnung vergrößert als Detail aus Figur1, wie der festgeschraubte Elektrodendeckel (12) mit seinem Deckelsteg (15) das mit dem iontophoretischen Wirkstoff (21) getränkte Wirkstoff-Pad (20) am Rand einklemmt und auf die nach oben gewölbte elektrisch leitende Elektrodenoberfläche (13) ganzflächig festklemmt und wie das Wirkstoff-Pad (20) über den Elektrodendeckel (12) herausragt, so dass bei der lontophoresebehandlung nur das Wirkstoff-Pad (20) die zu behandelnde Hautpartie des Patienten zur Wirkstoffübertragung berührt.

## Patentansprüche

1. lontophorese-Kaltkontaktelektrodensystem,
mit einem Elektrodenkörper (11) mit einer definiert kühlbaren Elektrodenoberfläche (13) zum Einbringen von iontophoretischen Wirkstoffen in die menschliche Haut und zum gleichzeitigen Kühlen der menschlichen Haut, um die elektrische Leitfähigkeit der Haut und damit das Einbringen der iontophoretischen Wirkstoffe in die Haut zu verbessern,
dass die Elektrodenoberfläche (13) wahlweise definiert gekühlt oder definiert erwärmt wird durch ein Peltier-Element (30), wobei die Erwärmung der Elektrodenoberfläche über das Umschalten der Polarität am Peltier-Element (30) erfolgt, oder durch ein Leitungssystem, an welches die Elektrodenoberfläche (13) angeschlossen ist, um die Elektrodenoberfläche (13) durch Vorbeileiten eines entsprechend gekühlten oder erwärmten Mittels zu erwärmen bzw. zu kühlen.

2. lontophorese-Kaltkontaktelektrodensystem nach Anspruch 1,
**dadurch gekennzeichnet,**
**dass** eine Kühltemperatur der Elektrodenoberfläche (13) durch einen integrierten Temperatursensor (36) erfasst und über eine Regelung auf eine Temperatur bis -5°C definiert wählbar gesteuert wird.

3. lontophorese-Kaltkontaktelektrodensystem nach Anspruch 1 oder Anspruch 2,
**dadurch gekennzeichnet,**
**dass** die Elektrodenoberfläche (13) an ein Leitungssystem angeschlossen ist, wobei das zum Erwärmen bzw. Kühlen der Elektrodenoberfläche benutzte Mittel ein Gas ist.

4. lontophorese-Kaltkontaktelektrodensystem nach einem der vorhergehenden Ansprüche,
**dadurch gekennzeichnet,**
**dass** die Elektrodenoberfläche (13) bei Bedarf definiert bis vorzugsweise auf +45°C erwärmbar ist.

5. lontophorese-Kaltkontaktelektrodensystem, nach einem der vorhergehenden Ansprüche,
**gekennzeichnet durch**
eine Klemmvorrichtung (12, 15) zum Fixieren von Wirkstoff-Pads (20), welche mit den iontophoretischen Wirkstoffen getränkt sind, auf der Elektrodenoberfläche (13).

6. lontophorese-Kaltkontaktelektrodensystem nach Anspruch 5,
**dadurch gekennzeichnet,**
**dass** die Klemmvorrichtung derart ausgeführt ist, dass auf dem Elektrodenkörper (11), mit einer nach außen gewölbten, elektrisch leitenden Elektrodenoberfläche (13), der aus einem porösen, nicht leitenden Material bestehende und mit dem iontophoretischem Wirkstoff getränkte Wirkstoff-Pad (20) durch einen Deckelsteg (15) eines oben offenen Elektrodendeckels (12) ganzflächig so auf der Elektrodenoberfläche (13) festgeklemmt wird, dass der Wirkstoff-Pad erhaben über den Elektrodenkörper herausragend fixiert wird und **dadurch** über die menschliche Haut geführt werden kann und einen lontophorese-Stromkreis zur einer an einem anderen Körperbereich angeschlossenen Groundelektrode schließt.

7. lontophorese-Kaltkontaktelektrodensystem nach Anspruch 5 oder Anspruch 6,
**dadurch gekennzeichnet,**
**dass** der Wirkstoff-Pad (20) aus einem flexiblen, porösen, an der Oberfläche aufgerauhten, nichtleitenden Material besteht und mit einem oder mehreren elektrisch leitenden, in Flüssigkeit oder Gel oder Creme gelösten iontophoretischen Wirkstoffen getränkt ist.

8. lontophorese-Kaltkontaktelektrodensystem nach einem der Ansprüche 5-7,
**dadurch gekennzeichnet,**
**dass** der Wirkstoff-Pad (20) mit medizinischen Wirkstoffen, kosmetischen Wirkstoffen oder nahrungsergänzenden Wirkstoffen getränkt ist, die für das Einbringen in die menschliche Haut bestimmt sind.

9. lontophorese-Kaltkontaktelektrodensystem nach einem der Ansprüche 5-8,
**dadurch gekennzeichnet,**
**dass** der Wirkstoff-Pad (20) ein Wirkstoff-Einwegpad ist.

10. Iontophorese-Kaltkontaktelektrodensystem nach einem der vorhergehenden Ansprüche,
**dadurch gekennzeichnet,**
**dass** die Elektrodenoberfläche (13) zum Zwecke der Abrasion von verhornten Zellen der oberen Hautschicht gleichzeitig zum Kühlen und Einbringen der iontophoretischen Wirkstoffe in die Haut ausgestaltet ist.
